# EUROPEAN PATENT APPLICATION

(11) **EP 4 302 828 A2**
(43) Date of publication of application: **10.01.2024**
(21) Application number: 23196788.6
(22) Date of filing: 04.07.2019
(51) Int. Cl.: A61P 25/06

(54) **COMBINATION THERAPY WITH CGRP ANTAGONISTS AND CLOSTRIDIAL DERIVATIVES**

(30) Priority: 05.07.2018 US 201862694358 P
(62) Divisional of application: 19769212.2
(71) Applicant: Allergan Pharmaceuticals International Limited, Dublin, D17 E400 (IE)
(72) Inventor: Blumenfeld, Andrew M., Del Mar, California, 92014 (US); Brin, Mitchell F., Newport Beach, CA, 92660 (US)
(74) Representative: Hoffmann Eitle

(57) **Abstract**

Methods for treating headache, migraine and related symptoms by administering a CGRP antagonist and a Clostridial derivative are described.

## Description

### FIELD

The application is related to medicaments and methods for treating migraine, headache and related symptoms. The present application is directed to a medicament functioning as a calcitonin-gene-related-peptide (hereafter referred to as CGRP) antagonist administered in combination with a clostridial derivative, such as a botulinum toxin, for treating headache, migraine and related symptoms.

### BACKGROUND

Migraine is a common neurological disorder that affects up to 16 % of the adult population in Western countries. It is characterized by attacks, often of disabling headache, which can be associated with sensory, visual, or language (aura), associated symptoms such as nausea and/or vomiting, phonophobia and photophobia, as well as dizziness and cognitive symptoms. Although the triptan group of drugs provides effective relief from acute migraine attacks for many patients, a substantial number (up to 40% in the case of oral triptans) of affected individuals are unresponsive. (Edvinsson L. Br J Clin Pharmacol. 80:2 193-199, 2015) Migraine is a highly disabling neurological condition, and preventative treatment still remains problematic, due to aspecificity of the majority of the currently available prophylactic drugs. (Adele M., et. al., Journal of Pain Research 2017: 10 2751-2760, 2018). During a migraine attack the trigeminal ganglion and trigeminal nerve endings secrete many neuropeptides that are involved in migraine pathophysiology. One of these neuropeptides is Calcitonin-gene-related peptide (CGRP), which plays a crucial role in migraine pathophysiology and several agents aimed at blocking its activity are currently being developed for the treatment of migraine, including a class of compounds knows as gepants and monoclonal antibodies (mAbs) against CGRP. Erenumab is a new promising candidate for the treatment of episodic migraine. (Adele M., et. al.) Erenumab is a fully human monoclonal antibody that binds to the CGRP receptor. (Goadsby P. J. et. al., N Engl J Med 2017; 377:2123-2132). OnabotulinumtoxinA (BOTOX) is an FDA-approved treatment for chronic migraine headaches. (Janis JE, et. al., Plast Reconstr Surg Glob Open 2017;5:e1270). While a variety of treatment options are available, many are ineffective and have undesirable side effects. There is a need for new and improved methods for the treatment and prevention of migraine and associated symptoms.

### SUMMARY

The application provides methods for treating, preventing, alleviating or reducing the frequency of occurrence of migraine, headache, or a symptom of migraine in patients by the use antagonists of calcitonin gene-related peptide (CGRP-antagonists), wherein the patient is concurrently undergoing treatment with a clostridial derivative. In some embodiments, the patient is administered CGRP-antagonist, and, a clostridial derivative. In some embodiments, the clostridial derivative is a recombinant clostridial toxin, a recombinant modified clostridial toxin, fragments of botulinum toxin, or targeted exocytosis modulators (TEMs), or combinations thereof. In some embodiments, the CGRP-antagonist is ubrogepant, atogepant, or a pharmaceutically acceptable salt, ester or prodrug thereof. In some embodiments, the CGRP-antagonist is an antibody is selected from galcanezumab, fremanezumab, eptinezumab, and erenumab.

### DESCRIPTION

The application provides methods for treating, preventing, alleviating or reducing the frequency of occurrence of migraine, headache, or a symptom of migraine in patients by the use antagonists of calcitonin gene-related peptide (CGRP-antagonists), wherein the patient is concurrently undergoing treatment with a clostridial derivative. In some embodiments, the clostridial derivative is a botulinum toxin. In one embodiment, the clostridial derivative is onabotulinumtoxinA. In some embodiments, the methods relate to administering to the patient a CGRP-antagonist, and a botulinum toxin, preferably onabotulinumtoxinA. In embodiments, the combination therapy of botulinum toxin and a CGRP antagonist reduces the frequency, severity and/or duration of migraine headache in patients in need thereof. In some embodiments the combination therapy of botulinum toxin and a CGRP antagonist reduces the associated symptoms of migraine such as nausea, vomiting, photophobia, phonophobia, and aura symptoms.

In some embodiments, the combination therapy is administered to the patient at a dose sufficient to reduce the number of monthly migraine headache days experienced by the patient as compared to the number of monthly migraine headache days prior to treatment or the number of monthly migraine headache days experienced by a patient receiving the treatment with CGRP antagonist alone or botulinum toxin alone.

In some embodiments, the headache is a migraine headache, a cluster headache, a hemicrania continua headache, a new daily persistent headache, a chronic daily headache, a tension headache, a chronic tension headache, or a combination thereof. In some embodiments, the migraine is migraine without aura. In some embodiments, the migraine is menstrual migraine, familial hemiplegic migraine, migraine with brainstem aura, vestibular migraine, migraine triggered by a traumatic brain injury or chronic migraine.

In some embodiments, the CGRP-antagonist is an anti-calcitonin gene-related peptide receptor antibody (anti-CGRP antibody) or antigen-binding fragment thereof. For example, the antibody can be selected from galcanezumab, fremanezumab, eptinezumab or erenumab. In some embodiments, the anti-CGRP antibody or fragment thereof is administered at a dosage that is about 20% or 30% or 40% or 50% or 60% or 70% or 80% lower than the recommended dosage for the anti-CGRP antibody monotherapy. In some embodiments, the anti-CGRP antibody or antigen-binding fragment thereof is administered to a peripheral nerve, a cranial nerve, or combinations thereof. CGRP-antagonists can be administered by any method that allows the antagonist to reach the intended targets.

For example, erenumab can be administered weekly, biweekly, monthly, every two months, every three months, every four months, every five months or every six months at a dosage of about 5 mg to about 500 mg.

Erenumab can be administered parenterally, subcutaneously or by peripheral administration. (Brauser D., Phase 3 STRIVE and ARISE Trials Show Efficacy, Safety for Erenumab in Migraine Prevention, Medscape Medical News, 2017) In one embodiment, erenumab is administered to trigeminal nerves.

In some embodiments, erenumab can be administered to the patient over the course of a set treatment period or indefinitely. (U.S. Patent Publication No. 20160311913) The treatment period can begin upon administration of a first dose of erenumab and continue while they have symptoms. The combination therapy with botulinum toxin includes administration of botulinum toxin prior to, during or after the treatment period with erenumab. The treatment period can vary and in some embodiments, lasts only one day where the antibody is administered, and in some embodiments, can continue indefinitely while the patient continues to suffer from headache or migraine. The treatment period may comprise from about 1 month to about 36 months, such as about 2 months, about 3 months, about 4 months, about 5 months, about 6 months, about 7 months, about 8 months, about 9 months, about 10 months, about 11 months, about 12 months, about 13 months, about 14 months, about 15 months, about 18 months, about 21 months, about 24 months, about 27 months, about 30 months, or about 33 months. In some embodiments, the treatment period is about 6 months. In other embodiments, the treatment period is about 7 months. In yet other embodiments, the treatment period is about 12 months. In certain embodiments, the treatment period can be longer than 36 months, such as 48 or 60 or 64 months or more. In some embodiment, erenumab is administered in a pharmaceutical composition comprising a buffer (preferably an acetate buffer), a surfactant (preferably polysorbate 20 or polysorbate 80), and a stabilizing agent (preferably sucrose). In one particular embodiment, the treatment period is at least about 6 months and produces a statistically significant reduction in the frequency, duration, or severity of migraine headache in the patient as compared to patients treated with erenumab or botulinum toxin alone.

In one embodiment, erenumab can be administered subcutaneously at a dose of about 5 mg to about 500 mg every one, two, three, four, five, six, seven, eight, nine or ten weeks.

In one embodiment, erenumab can be administered subcutaneously at a dose of about 10 mg to about 200 mg every one, two, three, four, five, six, seven, eight, nine or ten weeks.

In one embodiment, erenumab can be administered subcutaneously at a dose of about 25 mg to about 150 mg every one, two, three, four, five, six, seven, eight, nine or ten weeks.

In one embodiment, erenumab can be administered subcutaneously at a dose of about 90 mg to about 120 mg every one, two, three, four, five, six, seven, eight, nine or ten weeks.

In one embodiment, erenumab can be administered subcutaneously at a dose of about 50 mg to about 60 mg every one, two, three, four, five, six, seven, eight, nine or ten weeks.

In one embodiment, erenumab can be administered subcutaneously at a dose of about 70 mg every one, two, three, four, five, six, seven, eight, nine or ten weeks.

In one embodiment, erenumab can be administered subcutaneously at a dose of about 140 mg every one, two, three, four, five, six, seven, eight, nine or ten weeks.

In one embodiment, erenumab can be administered subcutaneously at a monthly dose of about 140 mg.

In one embodiment, erenumab can be administered subcutaneously at a monthly dose of about 70 mg.

In one embodiment, erenumab can be administered subcutaneously at a dose of about 140 mg every two months.

In one embodiment, erenumab can be administered subcutaneously at a dose of about 70 mg every two months.

In one embodiment, erenumab can be administered subcutaneously at a dose of about 140 mg every three months.

In one embodiment, erenumab can be administered subcutaneously at a dose of about 70 mg every three months.

In one embodiment, an anti-CGRP antibody galcanezumab can be administered weekly, biweekly, monthly, every two months, every three months, every four months, every five months or every six months at a dosage of about 5 mg to about 500 mg.

In some embodiments, galcanezumab can be administered to the patient over the course of a set treatment period. The treatment period can begin upon administration of a first dose galcanezumab and ends upon administration of a final dose of galcanezumab. The combination therapy with botulinum toxin includes administration of botulinum toxin prior to, during or after the treatment period with galcanezumab. The treatment period may comprise from about 1 month to about 36 months, such as about 2 months, about 3 months, about 4 months, about 5 months, about 6 months, about 7 months, about 8 months, about 9 months, about 10 months, about 11 months, about 12 months, about 13 months, about 14 months, about 15 months, about 18 months, about 21 months, about 24 months, about 27 months, about 30 months, or about 33 months. In some embodiments, the treatment period is about 6 months. In other embodiments, the treatment period is about 7 months. In yet other embodiments, the treatment period is about 12 months. In certain embodiments, the treatment period can be longer than 36 months, such as 48 or 60 or 64 months or more. In one particular embodiment, the treatment period is at least about 6 months and produces a statistically significant reduction in the frequency, duration, or severity of migraine headache in the patient as compared to patients treated with galcanezumab or botulinum toxin alone.

In one embodiment, galcanezumab is administered subcutaneously at a dose of about 10 mg to about 500 mg every one, two, three, four, five, six, seven, eight, nine or ten weeks.

In one embodiment, galcanezumab is administered subcutaneously at a dose of about 50 mg to about 300 mg every one, two, three, four, five, six, seven, eight, nine or ten weeks.

In one embodiment, galcanezumab is administered subcutaneously at a dose of about 75 mg to about 250 mg every one, two, three, four, five, six, seven, eight, nine or ten weeks.

In one embodiment, galcanezumab is administered subcutaneously at a dose of about 75 mg to about 100 mg every one, two, three, four, five, six, seven, eight, nine or ten weeks.

In one embodiment, galcanezumab is administered subcutaneously at a dose of about 150 mg to about 220 mg every one, two, three, four, five, six, seven, eight, nine or ten weeks.

In one embodiment, galcanezumab is administered subcutaneously at a dose of about 120 mg every one, two, three, four, five, six, seven, eight, nine or ten weeks.

In one embodiment, galcanezumab is administered subcutaneously at a dose of about 240 mg every one, two, three, four, five, six, seven, eight, nine or ten weeks.

In one embodiment, galcanezumab is administered subcutaneously at a monthly dose of about 240 mg.

In one embodiment, galcanezumab is administered subcutaneously at a monthly dose of about 120 mg.

In one embodiment, galcanezumab is administered subcutaneously at a dose of about 240 mg every two months.

In one embodiment, galcanezumab is administered subcutaneously at a dose of about 120 mg every two months.

In one embodiment, galcanezumab is administered subcutaneously at a dose of about 240 mg every three months.

In one embodiment, galcanezumab is administered subcutaneously at a dose of about 120 mg every three months.

In some embodiments, fremanezumab can be administered to the patient over the course of a set treatment period. (Silberstein, S.D., et.al., N Engl J Med 2017;377:2113-22.) The treatment period can begin upon administration of a first dose fremanezumab and ends upon administration of a final dose of fremanezumab. The combination therapy with botulinum toxin includes administration of botulinum toxin prior to, during or after the treatment period with fremanezumab. The treatment period may comprise from about 1 month to about 36 months, such as about 2 months, about 3 months, about 4 months, about 5 months, about 6 months, about 7 months, about 8 months, about 9 months, about 10 months, about 11 months, about 12 months, about 13 months, about 14 months, about 15 months, about 18 months, about 21 months, about 24 months, about 27 months, about 30 months, or about 33 months. In some embodiments, the treatment period is about 6 months. In other embodiments, the treatment period is about 7 months. In yet other embodiments, the treatment period is about 12 months. In certain embodiments, the treatment period can be longer than 36 months, such as 48 or 60 or 64 months or more. In one particular embodiment, the treatment period is at least about 6 months and produces a statistically significant reduction in the frequency, duration, or severity of migraine headache in the patient as compared to patients treated with fremanezumab or botulinum toxin alone.

In one embodiment, fremanezumab is administered subcutaneously at a dose of about 100 mg to about 1000 mg every one, two, three, four, five, six, seven, eight, nine or ten weeks.

In one embodiment, fremanezumab is administered subcutaneously at a dose of about 150 mg to about 700 mg every one, two, three, four, five, six, seven, eight, nine or ten weeks.

In one embodiment, fremanezumab is administered subcutaneously at a dose of about 150 mg to about 500 mg every one, two, three, four, five, six, seven, eight, nine or ten weeks.

In one embodiment, fremanezumab is administered subcutaneously at a dose of about 150 mg to about 200 mg every one, two, three, four, five, six, seven, eight, nine or ten weeks.

In one embodiment, fremanezumab is administered subcutaneously at a dose of about 150 mg to about 500 mg every one, two, three, four, five, six, seven, eight, nine or ten weeks.

In one embodiment, fremanezumab is administered subcutaneously at a dose of about 225 mg every one, two, three, four, five, six, seven, eight, nine or ten weeks.

In one embodiment, fremanezumab is administered subcutaneously at a dose of about 450 mg every one, two, three, four, five, six, seven, eight, nine or ten weeks.

In one embodiment, fremanezumab is administered subcutaneously at a dose of about 675 mg every one, two, three, four, five, six, seven, eight, nine or ten weeks.

In one embodiment, fremanezumab is administered subcutaneously at a monthly dose of about 225 mg.

In one embodiment, fremanezumab is administered subcutaneously at a monthly dose of about 450 mg.

In one embodiment, fremanezumab is administered subcutaneously at a monthly dose of about 675 mg.

In one embodiment, fremanezumab is administered subcutaneously at a dose of about 225 mg every two months.

In one embodiment, fremanezumab is administered subcutaneously at a dose of about 450 mg every two months.

In one embodiment, fremanezumab is administered subcutaneously at a dose of about 225 mg every three months.

In one embodiment, fremanezumab is administered subcutaneously at a dose of about 450 mg every three months.

In one embodiment, fremanezumab is administered subcutaneously at a dose of about 675 mg every three months.

In some embodiments, eptinezumab can be administered to the patient over the course of a set treatment period. The treatment period can begin upon administration of a first dose eptinezumab and ends upon administration of a final dose of eptinezumab. The combination therapy with botulinum toxin includes administration of botulinum toxin prior to, during or after the treatment period with eptinezumab. The treatment period may comprise from about 1 month to about 36 months, such as about 2 months, about 3 months, about 4 months, about 5 months, about 6 months, about 7 months, about 8 months, about 9 months, about 10 months, about 11 months, about 12 months, about 13 months, about 14 months, about 15 months, about 18 months, about 21 months, about 24 months, about 27 months, about 30 months, or about 33 months. In some embodiments, the treatment period is about 6 months. In other embodiments, the treatment period is about 7 months. In yet other embodiments, the treatment period is about 12 months. In certain embodiments, the treatment period can be longer than 36 months, such as 48 or 60 or 64 months or more. In one particular embodiment, the treatment period is at least about 6 months and produces a statistically significant reduction in the frequency, duration, or severity of migraine headache in the patient as compared to patients treated with eptinezumab or botulinum toxin alone.

In one embodiment, eptinezumab is administered subcutaneously at a dose of about 50 mg to about 1000 mg every one, two, three, four, five, six, seven, eight, nine or ten weeks.

In one embodiment, eptinezumab is administered subcutaneously at a dose of about 100 mg to about 700 mg every one, two, three, four, five, six, seven, eight, nine or ten weeks.

In one embodiment, eptinezumab is administered subcutaneously at a dose of about 200 mg to about 500 mg every one, two, three, four, five, six, seven, eight, nine or ten weeks.

In one embodiment, eptinezumab is administered subcutaneously at a dose of about 250 mg to about 350 mg every one, two, three, four, five, six, seven, eight, nine or ten weeks.

In one embodiment, eptinezumab is administered subcutaneously at a dose of about 300 mg every one, two, three, four, five, six, seven, eight, nine or ten weeks.

In one embodiment, eptinezumab is administered subcutaneously at a monthly dose of about 100 mg.

In one embodiment, eptinezumab is administered subcutaneously at a monthly dose of about 200 mg.

In one embodiment, eptinezumab is administered subcutaneously at a monthly dose of about 300 mg.

In one embodiment, eptinezumab is administered subcutaneously at a dose of about 100 mg every two months.

In one embodiment, eptinezumab is administered subcutaneously at a dose of about 200 mg every two months.

In one embodiment, eptinezumab is administered subcutaneously at a dose of about 300 mg every two months.

In one embodiment, eptinezumab is administered subcutaneously at a dose of about 100 mg every three months.

In one embodiment, eptinezumab is administered subcutaneously at a dose of about 200 mg every three months.

In one embodiment, eptinezumab is administered subcutaneously at a dose of about 300 mg every three months.

In some embodiments, an antagonist of CGRP receptor can be administered in combination with a botulinum toxin. Preferably, the CGRP antagonist is selected from ubrogepant, atogepant, rimegepant or a pharmaceutically acceptable salt thereof.

In some embodiments, ubrogepant can be administered to the patient over the course of a set treatment period or indefinitely while the patient needs treatment. The treatment can be given over the life of the patent's disease continuously or intermittently as needed. In some embodiments, the treatment period includes a single administration of ubrogepant to a patient undergoing treatment with a botulinum toxin. In some embodiments, the treatment period continues where the patient is administered ubrogepant continuously or intermittently for a year or more. The treatment period can begin upon administration of a first dose of ubrogepant and continue until the patient is administered ubrogepant on a regular or intermittent basis. The combination therapy with botulinum toxin includes administration of botulinum toxin prior to, during or after the treatment period with ubrogepant. The treatment period may comprise from about 1 month to about 36 months, such as about 2 months, about 3 months, about 4 months, about 5 months, about 6 months, about 7 months, about 8 months, about 9 months, about 10 months, about 11 months, about 12 months, about 13 months, about 14 months, about 15 months, about 18 months, about 21 months, about 24 months, about 27 months, about 30 months, or about 33 months. In some embodiments, the treatment period is about 6 months. In other embodiments, the treatment period is about 7 months. In yet other embodiments, the treatment period is about 12 months. In certain embodiments, the treatment period can be longer than 36 months, such as 48 or 60 or 64 months or more. In one particular embodiment, the treatment period is at least about 6 months and produces a statistically significant reduction in the frequency, duration, or severity of migraine headache in the patient as compared to patients treated with ubrogepant or botulinum toxin alone.

In some embodiments, ubrogepant is administered at an oral dose of about 5 to about 500 mg once, twice or three times a day.

In some embodiments, ubrogepant is administered at an oral dose of about 25 mg once, twice or three times a day.

In some embodiments, ubrogepant is administered at an oral dose of about 50 mg once, twice or three times a day.

In some embodiments, ubrogepant is administered at an oral dose of about 100 mg once, twice or three times a day.

In some embodiments, ubrogepant is administered at an oral dose of about 200 mg once, twice or three times a day.

In some embodiments, atogepant can be administered to the patient over the course of a set treatment period or indefinitely while the patient needs treatment. The treatment can be given over the life of the patent's disease continuously or intermittently as needed. In some embodiments, the treatment period includes a single administration of atogepant to a patient undergoing treatment with a botulinum toxin. In some embodiments, the treatment period continues where the patient is administered atogepant continuously or intermittently for a year or more. The treatment period can begin upon administration of a first dose atogepant and continue until the patient is administered atogepant on a regular or intermittent basis. The combination therapy with botulinum toxin includes administration of botulinum toxin prior to, during or after the treatment period with atogepant. The treatment period may comprise from about 1 month to about 36 months, such as about 2 months, about 3 months, about 4 months, about 5 months, about 6 months, about 7 months, about 8 months, about 9 months, about 10 months, about 11 months, about 12 months, about 13 months, about 14 months, about 15 months, about 18 months, about 21 months, about 24 months, about 27 months, about 30 months, or about 33 months. In some embodiments, the treatment period is about 6 months. In other embodiments, the treatment period is about 7 months. In yet other embodiments, the treatment period is about 12 months. In certain embodiments, the treatment period can be longer than 36 months, such as 48 or 60 or 64 months or more. In one particular embodiment, the treatment period is at least about 6 months and produces a statistically significant reduction in the frequency, duration, or severity of migraine headache in the patient as compared to patients treated with atogepant or botulinum toxin alone.

In some embodiments, atogepant is administered at an oral dose of about 5 to about 500 mg once, twice or three times a day.

In some embodiments, atogepant is administered at an oral dose of about 25 mg once, twice or three times a day.

In some embodiments, atogepant is administered at an oral dose of about 50 mg once, twice or three times a day.

In some embodiments, atogepant is administered at an oral dose of about 100 mg once, twice or three times a day.

In some embodiments, atogepant is administered at an oral dose of about 200 mg once, twice or three times a day.

In some embodiments, rimegepant can be administered to the patient over the course of a set treatment period or indefinitely while the patient needs treatment. The treatment can be given over the life of the patent's disease continuously or intermittently as needed. In some embodiments, the treatment period includes a single administration of rimegepant to a patient undergoing treatment with a botulinum toxin. In some embodiments, the treatment period continues where the patient is administered rimegepant continuously or intermittently for a year or more. The treatment period can begin upon administration of a first dose rimegepant and continue until the patient is administered rimegepant on a regular or intermittent basis. The combination therapy with botulinum toxin includes administration of botulinum toxin prior to, during or after the treatment period with rimegepant. The treatment period may comprise from about 1 month to about 36 months, such as about 2 months, about 3 months, about 4 months, about 5 months, about 6 months, about 7 months, about 8 months, about 9 months, about 10 months, about 11 months, about 12 months, about 13 months, about 14 months, about 15 months, about 18 months, about 21 months, about 24 months, about 27 months, about 30 months, or about 33 months. In some embodiments, the treatment period is about 6 months. In other embodiments, the treatment period is about 7 months. In yet other embodiments, the treatment period is about 12 months. In certain embodiments, the treatment period can be longer than 36 months, such as 48 or 60 or 64 months or more. In one particular embodiment, the treatment period is at least about 6 months and produces a statistically significant reduction in the frequency, duration, or severity of migraine headache in the patient as compared to patients treated with rimegepant or botulinum toxin alone.

In some embodiments, rimegepant is administered at an oral dose of about 5 to about 500 mg once, twice or three times a day.

In some embodiments, rimegepant is administered at an oral dose of about 25 mg once, twice or three times a day.

In some embodiments, rimegepant is administered at an oral dose of about 50 mg once, twice or three times a day.

In some embodiments, rimegepant is administered at an oral dose of about 100 mg once, twice or three times a day.

In some embodiments, rimegepant is administered at an oral dose of about 200 mg once, twice or three times a day.

In some embodiments, rimegepant is administered at an oral dose of about 5 to about 500 mg once, twice or three times a day.

In some embodiments, rimegepant is administered at an oral dose of about 25 mg once, twice or three times a day.

In some embodiments, rimegepant is administered at an oral dose of about 50 mg once, twice or three times a day.

In some embodiments, rimegepant is administered at an oral dose of about 100 mg once, twice or three times a day.

In some embodiments, rimegepant is administered at an oral dose of about 200 mg once, twice or three times a day.

In some embodiments, the combination therapy with a clostridial derivative, such as a botulinum toxin, reduces the frequency, severity and/or duration of migraine headache in patients in need thereof. In some embodiments, the combination therapy reduces the frequency of one or more symptoms or side effects of migraine; for example, sinusitis, nausea or nasopharangytis, in comparison with a patient undergoing treatment with CGRP-antagonist or botulinum toxin treatment alone. In some embodiments, the frequency of symptoms associated with one or more stages of migraine selected from prodrome, aura, headache or postdrome is reduced. For example, one or more frequency of symptoms associated with prodrome stage of migraine selected from photophobia, appetite changes, cognition and concentration difficulties, cold extremities, diarrhea or other bowel changes, excitement or irritability, fatigue, frequent urination, memory changes, weakness, yawning and stretching is reduced. In some embodiments, one or more frequency of symptoms associated with aura stage of migraine selected from seeing bright spots or flashes of light, vision loss, seeing dark spots, tingling sensations, speech problems, aphasia, and tinnitus is reduced. In some embodiments, one or more frequency of symptoms associated with attack stage of migraine selected from photophobia, gastric stasis, pulsating or throbbing pain on one or both sides of the head, extreme sensitivity to light, sounds, or smells, worsening pain during physical activity, nausea and vomiting, abdominal pain or heartburn, loss of appetite, lightheadedness, blurred vision, and fainting is reduced.

In some embodiments, the combination therapy with CGRP-antagonists and a clostridial derivative described herein is administered to a patient undergoing treatment with one or more additional medications for the treatment of headache. For example, the patient can be undergoing treatment with one or more additional medications selected from opioid analgesics, Cox-2 inhibitors, barbiturates, sodium channel modulators, potassium channel modulators, calcium channel blockers, local anesthetics, monoamine oxidase inhibitors, leukotriene LTD4 receptor blocker, substance P antagonists, 5-HT3 antagonists and NMDA antagonists. For example, the additional medication can be selected from aspirin, ibuprofen, naproxen, acetaminophen, diclofenac, flurbiprofen, meclofenamate, isometheptene, indomethacin; codeine, morphine, hydrocodone, acetyldihydrocodeine, oxycodone, oxymorphone, papaverine, fentanyl, alfentanil, sufentanil, remifentanyl, tramadol, prochlorperazine, celecoxib, rofecoxib, meloxicam, piroxicam, JTE-522, L-745,337, NS398, deracoxib, valdecoxib, iumiracoxib, etoricoxib, parecoxib, 4-(4-cyclohexyl-2-methyloxazol-5-yl)-2 fluorobenzenesulfonamide, (2-(3,5-difluorophenyl)-3-(4-(methylsulfonyl)phenyl)-2 cyclopenten-1-one, N-[2-(cyclohexyloxy)-4-nitrophenyl]methanesulfonamide, 2-(3,4 difluorophenyl)-4-(3-hydroxy-3-methylbutoxy)-5-[4-(methylsulfonyl) phenyl]-3(2H) pyridazinone, 2-[(2,4-dichloro-6-methylphenyl) amino]-5-ethyl-benzeneacetic acid, (3Z) 3-[(4-chlorophenyl) [4-(methylsulfonyl)phenyl] methylene]dihydro-2(3H)-furanone, (S)-6,8-dichloro-2-(trifluoromethyl)-2H-1-benzopyran-3-carboxylic acid, amobarbital, butalbital, cyclobarbital, pentobarbital, allobarbital, methylphenobarbital, phenobarbital, secobarbital, vinylbital, verapamil, ciltiazem, Nifedipine, lidocaine, tetracaine, prilocaine, bupivicaine, mepivacaine, etidocaine, procaine, benzocaine, phehelzine, isocarboxazid, dichloralphenazone, nimopidine, metoclopramide, capsaicin receptor agonists, captopril, tiospirone, a steroid, caffeine, metoclopramide, domperidone, scopolamine, dimenhydrinate, diphenhydramine, hydroxyzine, diazepam, lorazepam, chlorpromazine, methotrimeprazine, perphenazine, prochlorperazine, promethazine, trifluoperazine, triflupromazine, benzquinamide, bismuth subsalicylate, buclizine, cinnarizine, cyclizine, diphenidol, dolasetron, domperidone, dronabinol, droperidol, haloperidol, metoclopramide, nabilone, thiethylperazine, trimethobenzemide, and eziopitant, Meclizine, domperidone, ondansetron, tropisetron granisetron dolasetron, hydrodolasetron, palonosetron, alosetron, cilansetron, cisapride, renzapride metoclopramide, galanolactone, phencyclidine, ketamine, dextromethorphan, and isomers, pharmaceutically acceptable salts, esters, conjugates, or prodrugs thereof.

In some embodiments, the clostridial derivative is onabotulinumtoxinA and is administered at a dose of about 1 unit, about 2 units, about 3 units, about 4 units, about 5 units, about 6 units, about 7 units, about 8 units, about 9 units or about 10 units. The frequency of administration can be once every one, two, three, four, five, six, seven, eight, nine, ten, eleven, twelve, thirteen, fourteen, fifteen or sixteen weeks.

In some embodiments, onabotulinumtoxinA is administered at a dose of about 10 unit, about 15 units, about 20 units, about 25 units, about 30 units, about 40 units, about 45 units, about 50 units, about 55 units or about 60 units. The frequency of administration can be once every one, two, three, four, five, six, seven, eight, nine, ten, eleven, twelve, thirteen, fourteen, fifteen or sixteen weeks.

In some embodiments, the clostridial derivative is onabotulinumtoxinA and is administered at a dose of about of about 25 unit, about 50 units, about 75 units, about 100 units, about 125 units, about 150 units, about 175 units, about 200 units, about 225 units or about 250 units every one, two, three, four, five, six, seven, eight, nine, ten, eleven, twelve, thirteen, fourteen, fifteen or sixteen weeks.

In some embodiments, the clostridial derivative is onabotulinumtoxinA and is administered at a dose of about dose of about 1 to about 1,000 units. The frequency of administration can be once every one, two, three, four, five, six, seven, eight, nine, ten, eleven, twelve, thirteen, fourteen, fifteen, sixteen, seventeen, eighteen, nineteen or twenty weeks, or more. In some embodiments, the frequency of administration can be once every one, two, three, four, five, six, seven, eight, nine, ten, eleven, twelve, thirteen, fourteen, fifteen, sixteen, seventeen, eighteen months, or more. In some embodiments, the clostridial derivative is onabotulinumtoxinA and is administered at a dose of about 1 to about 100 units every one, two, three, four, five, six, seven, eight, nine, ten, eleven, twelve, thirteen, fourteen, fifteen or sixteen weeks.

In some embodiments, the clostridial derivative is onabotulinumtoxinA and is administered at a dose of about 10 to about 50 units. The frequency of administration can be once every one, two, three, four, five, six, seven, eight, nine, ten, eleven, twelve, thirteen, fourteen, fifteen or sixteen weeks.

The clostridial derivative can be administered by many different methods including peripheral, parenteral, subcutaneous, intravenous, intraperitoneal, intracerebral, intralesional, intramuscular, intraocular, intraarterial interstitial infusion and implanted delivery device. In some embodiments, the clostridial derivative is administered to a nerve. In some embodiments, the clostridial derivative is administered to a suture line. In one embodiment, the clostridial derivative is onabotulinumtoxinA.

In some embodiments, onabotulinumtoxinA is administered parenterally.

In some embodiments, onabotulinumtoxinA is administered topically.

In some embodiments, onabotulinumtoxinA is administered subcutaneously or intramuscularly.

In some embodiments, onabotulinumtoxinA is administered intradermally.

In some embodiments, onabotulinumtoxinA is administered subcutaneously once every month or two months.

In some embodiments, onabotulinumtoxinA is administered at a dose of about 155 units.

The effective amount of the clostridial derivative can be measured in mass units (e.g. in ng or mg). The effective dose in weight units can be determined based on the intended effect. For example, the effective weight can be determined based on the amount of clostridial derivative required to have a therapeutic effect on the muscle or a sensory effect. In some embodiments, the clostridial derivative can be administered at a dose of about 0.001 ng to about 1000 ng, preferably about 0.001 ng to about 500 ng, preferably about 0.01 ng to about 250 ng, preferably about 0.1 ng to about 150 ng, preferably about 1 ng to about 100 ng, preferably about 1 ng to about 10 ng. For example, onabotulinumtoxinA can be administered at a dose of about 1 ng, 2 ng, 3 ng, 4 ng, 5 ng, 6 ng, 7 ng, 8 ng, 9 ng or 10 ng.

In some embodiments, the CGRP-antagonist can be administered orally, sublingually, transdermally, subcutaneously, intravenously, intradermally or intramuscularly.

In one embodiment, the CGRP-antagonist can be administered intravenously. The intravenous formulation can contain a tonicity modifier to avoid crenation or hemolysis of red blood cells, and/or to mitigate or avoid pain and discomfort to the patient. Preferably, the formulation to be administered to the patient has an effective osmotic pressure that is approximately the same as that of the blood of the patient. Tonicity modifiers can be non-ionic tonicity modifiers such as glycerol, sorbitol, mannitol, sucrose, propylene glycol or dextrose, or a mixture thereof. Preferably the non-ionic tonicity modifier is dextrose, sucrose or mannitol, or a mixture thereof. Aqueous pharmaceutical formulations for intravenous administration generally can have a pH of from 3 to 9.

Stable liquid or solid pharmaceutical composition comprising a clostridial toxin derivative, a disaccharide, a surfactant and an antioxidant can be used in combination with CGRP-antagonists.

CGRP is a member of the calcitonin family of peptides, which in human exists in two form, α-CGRP and β-CGRP. α-CGRP and β-CGRP vary by three amino acids, have similar activities and exhibit differential distribution. At least two CGRP receptor subtypes may also account for differential activities. CGRP is produced in both peripheral and central neurons, and released by the trigeminal nerve. CGRP has been shown to be a potent vasodilator in the periphery, where CGRP-containing neurons are closely associated with blood vessels. CGRP-mediated vasodilatation is also associated with neurogenic inflammation, as part of a cascade of events that results in extravasation of plasma and vasodilation of the microvasculature and is present in migraine. cGRP is released by sensory nerves, e.g. the trigeminal nerve, which innervates part of the skin of the face. The trigeminal nerve has three major branches, a number of smaller branches and is the great sensory nerve of the head and neck, carrying touch, temperature, pain, and proprioception (position sense) signals from the face and scalp to the brainstem. Trigeminal sensory fibers originate in the skin, course toward the trigeminal ganglion (a sensory nerve cell body), pass through the trigeminal ganglion, and travel within the trigeminal nerve to the sensory nucleus of the trigeminal nerve located in the brainstem.

The three major branches of the trigeminal nerve are the ophthalmic (V₁, sensory), maxillary (V₂, sensory) and mandibular (V₃, motor and sensory) branches. The large trigeminal sensory root and smaller trigeminal motor root leave the brainstem at the midlateral surface of pons. The sensory root terminates in the largest of the cranial nerve nuclei which extends from the pons all the way down into the second cervical level of the spinal cord. The sensory root joins the trigeminal or semilunar ganglion between the layers of the dura mater in a depression on the floor of the middle crania fossa. The trigeminal motor root originates from cells located in the masticator motor nucleus of trigeminal nerve located in the midpons of the brainstem. The motor root passes through the trigeminal ganglion and combines with the corresponding sensory root to become the mandibular nerve. It is distributed to the muscles of mastication, the mylohyoid muscle and the anterior belly of the digastric. The three sensory branches of the trigeminal nerve emanate from the ganglia to form the three branches of the trigeminal nerve. The ophthalmic and maxillary branches travel in the wall of the cavernous sinus just prior to leaving the cranium. The ophthalmic branch travels through the superior orbital fissure and passes through the orbit to reach the skin of the forehead and top of the head. The maxillary nerve enters the cranium through the foramen rotundum via the pterygopalatine fossa. Its sensory branches reach the pterygopalatine fossa via the inferior orbital fissure (and supply sensation to the face, cheek and upper teeth) and pterygopalatine canal (and supply sensation to the soft and hard palate, nasal cavity and pharynx). There are also meningeal sensory branches that enter the trigeminal ganglion within the cranium. The sensory part of the mandibular nerve is composed of branches that carry general sensory information from the mucous membranes of the mouth and cheek, anterior two-thirds of the tongue, lower teeth, skin of the lower jaw, side of the head and scalp and meninges of the anterior and middle cranial fossae.

The sensory nuclei of the trigeminal nerve are located within the brainstem, in the dorsolateral pons. The mesencephalic tract and the motor nucleus of the trigeminal nerve lie more medially. The superior cerebellar peduncle lies posteriorly. It is continuous inferiorly with the spinal nucleus of the trigeminal nerve that extends into the medulla. Superiorly, the sensory nuclei on each side are continuous with the mesencephalic nucleus.

Importantly, the sensory nuclei of the trigeminal nerve receive afferent (sensory input) fibers from: (1) the trigeminal nerve ophthalmic division (e.g. general sensation from supraorbital area, cornea, iris, ethmoid sinuses), (2) trigeminal nerve maxillary division (e.g. sensation from temple, cheek, oral cavity, upper pharynx), and (3) trigeminal nerve mandibular division (e.g. sensation from middle cranial fossa, inner cheek, anterior two thirds of the tongue, chin), (4) facial nerve (e.g. general sensation from external auditory meatus), (5) glossopharyngeal nerve (e.g. general sensation from middle ear, tonsils, oropharynx, posterior one third of the tongue), (6) vagus nerve (auricular, meningeal, internal laryngeal and recurrent laryngeal branches).

Thus, primary neurons in the trigeminal ganglion synapse on the main sensory trigeminal nucleus and on the spinal trigeminal nucleus in the brainstem. The spinal nucleus of the trigeminal system extends to the upper cervical spine, where connections with cervical dermatomes exist. These dermatomes are innervated by the cervical sensory rami and the occipital nerves, which have sensory branches from C2 to C5. The trigeminal nerve also innervates stretch receptors in the muscles of mastication. The cell bodies of these neurons are in the mesencephalic trigeminal nucleus in the midbrain and pons).

### Definitions

As used herein, the words or terms set forth below have the following definitions:

"About" or "approximately" as used herein means within an acceptable error range for the particular value as determined by one of ordinary skill in the art, which will depend in part on how the value is measured or determined, (*i.e*., the limitations of the measurement system). For example, "about" can mean within 1 or more than 1 standard deviations, per practice in the art. Where particular values are described in the application and statements, unless otherwise stated, the term "about" means within an acceptable error range for the particular value.

"Administration", or "to administer" means the step of giving (i.e. administering) a pharmaceutical composition to a subject, or alternatively a subject receiving a pharmaceutical composition. The combination therapy disclosed herein can be locally administered by various methods. For example, intramuscular, intradermal, subcutaneous administration, , intraperitoneal administration, topical (transdermal), instillation, and implantation (for example, of a slow-release device such as polymeric implant or miniosmotic pump) can all be appropriate routes of administration.

"Alleviating" means a reduction in the occurrence of a pain, of a headache, or of any symptom or cause of a condition or disorder. Thus, alleviating includes some reduction, significant reduction, near total reduction, and total reduction.

"Biological activity" describes the beneficial or adverse effects of a drug on living matter. When a drug is a complex chemical mixture, this activity is exerted by the substance's active ingredient but can be modified by the other constituents. Biological activity of a clostridial derivative such as a botulinum toxin can be assessed as potency or as toxicity by an *in vivo* LD₅₀ or ED₅₀ assay, or through an *in vitro* assay such as, for example, cell-based potency assays as described in U.S. 20100203559 and U.S. 20100233802.

"Botulinum toxin" means a neurotoxin produced by Clostridium botulinum, as well as a botulinum toxin, fragments, variants or chimeras thereof made recombinantly by a non-Clostridial species. The phrase "botulinum toxin", as used herein, encompasses Botulinum toxin serotype A (BoNT/A), Botulinum toxin serotype B (BoNT/B), Botulinum toxin serotype C (BoNT/C), Botulinum toxin serotype D (BoNT/D), Botulinum toxin serotype E (BoNT/E), Botulinum toxin serotype F (BoNT/F), Botulinum toxin serotype G (BoNT/G), Botulinum toxin serotype H (BoNT/H), Botulinum toxin serotype X (BoNT/X), Botulinum toxin serotype En (BoNT/En), and mosaic Botulinum toxins and/or their subtypes and any other types of subtypes thereof, or any re-engineered proteins, analogs, derivatives, homologs, parts, sub-parts, variants, or versions, in each case, of any of the foregoing. "Botulinum toxin", as used herein, also encompasses a "modified botulinum toxin". Further "botulinum toxin" as used herein also encompasses a botulinum toxin complex, (for example, the 300, 600 and 900kDa complexes), as well as the neurotoxic component of the botulinum toxin (150 kDa) that is unassociated with the complex proteins.

"CGRP", abbreviated for Calcitonin-Gene-Related-Peptide, as used herein encompasses any member of the calcitonin family, including any calcitonin gene related peptide and analogs, calcitonin, amylin, adrenomedullin and their analogs.

"CGRP antagonist" refers to any molecule that exhibits any one or more of the following characteristics: (a) bind to CGRP or CGRP-R and the binding results in a reduction or inhibition of CGRP activity; (b) block CGRP from binding to its receptor(s); (c) block or decrease CGRP receptor activation; (d) inhibit CGRP biological activity or downstream pathways mediated by CGRP signaling function; (e) increase clearance of CGRP; and (f) inhibit or reduce CGRP synthesis, production or release. CGRP antagonists include but are not limited to antibodies to CGRP, antibodies to the CGRP-R, small molecules that antagonize CGRP, and small molecules that antagonize CGRP-R.

"Clostridial derivative" refers to a molecule which contains any part of a clostridial toxin as defined herein. As used herein, the term "clostridial derivative" encompasses native or recombinant neurotoxins, recombinant modified toxins, fragments, chimeras and variants thereof, a Targeted Vesicular Exocytosis Modulator (TEM), or combinations thereof.

"Clostridial toxin" refers to any toxin produced by a Clostridial toxin strain that can execute the overall cellular mechanism whereby a Clostridial toxin intoxicates a cell and encompasses the binding of a Clostridial toxin to a low or high affinity Clostridial toxin receptor, the internalization of the toxin/receptor complex, the translocation of the Clostridial toxin light chain into the cytoplasm and the enzymatic modification of a Clostridial toxin substrate. Non-limiting examples of Clostridial toxins include a Botulinum toxin like BoNT/A, a BoNT/B, a BoNT/C₁, a BoNT/D, a BoNT/E, a BoNT/F, a BoNT/G, BoNT/H, a BoNT/En, BoNT/X, mosaic botulinum toxins, a Tetanus toxin (TeNT), a Baratii toxin (BaNT), and a Butyricum toxin (BuNT). The BoNT/C₂ cytotoxin and BoNT/C₃ cytotoxin, not being neurotoxins, are excluded from the term "Clostridial toxin." A Clostridial toxin disclosed herein includes, without limitation, naturally occurring Clostridial toxin variants, such as, *e*.*g*., Clostridial toxin isoforms and Clostridial toxin subtypes; non-naturally occurring Clostridial toxin variants, such as, *e.g.,* conservative Clostridial toxin variants, non-conservative Clostridial toxin variants, Clostridial toxin chimeric variants and active Clostridial toxin fragments thereof, or any combination thereof. A Clostridial toxin disclosed herein also includes a Clostridial toxin complex. As used herein, the term "Clostridial toxin complex" refers to a complex comprising a Clostridial toxin and non-toxin associated proteins (NAPs), such as, *e.g.,* a Botulinum toxin complex, a Tetanus toxin complex, a Baratii toxin complex, and a Butyricum toxin complex. Non-limiting examples of Clostridial toxin complexes include those produced by a *Clostridium botulinum,* such as, *e.g.,* a 900-kDa BoNT/A complex, a 500-kDa BoNT/A complex, a 300-kDa BoNT/A complex, a 500-kDa BoNT/B complex, a 500-kDa BoNT/C₁ complex, a 500-kDa BoNT/D complex, a 300-kDa BoNT/D complex, a 300-kDa BoNT/E complex, and a 300-kDa BoNT/F complex.

"Clostridial toxin active ingredient" refers to a molecule which contains any part of a clostridial toxin that exerts an effect upon or after administration to a subject or patient. As used herein, the term "clostridial toxin active ingredient" or "clostridial derivative" encompasses a Clostridial toxin complex comprising the approximately 150-kDa Clostridial toxin and other proteins collectively called non-toxin associated proteins (NAPs), the approximately 150-kDa Clostridial toxin alone, or a modified Clostridial toxin, such as, *e.g.,* a re-targeted Clostridial toxins.

"Combination therapy" refers to a treatment wherein a botulinum toxin and a CGRP antagonist are administered either simultaneously or sequentially, by a similar administration route or by different administration routes.

"Effective amount" as applied to the biologically active ingredient means that amount of the ingredient which is generally sufficient to effect a desired change in the subject. For example, where the desired effect is a reduction in duration or intensity of a headache, migraine and related symptoms, an effective amount of the ingredient is that amount which causes at least a substantial reduction in duration or intensity of the headache, migraine and related symptoms, and without resulting in significant toxicity.

"Intramuscular" or "intramuscularly" means into or within (as in administration or injection of a CGRP antagonist into) a muscle.

"Local administration" means direct administration of a pharmaceutical at or to the vicinity of a site on or within an animal body, at which site a biological effect of the pharmaceutical is desired, such as via, for example, intramuscular or intra- or subdermal injection or topical administration. Topical administration is a type of local administration in which a pharmaceutical agent is applied to a patient's skin.

"Modified botulinum toxin" means a botulinum toxin that has had at least one of its amino acids deleted, modified, or replaced, as compared to a native botulinum toxin. Additionally, the modified botulinum toxin can be a recombinantly produced neurotoxin, or a derivative or fragment of a recombinantly made neurotoxin. A modified botulinum toxin retains at least one biological activity of the native botulinum toxin, such as, the ability to bind to a botulinum toxin receptor, or the ability to inhibit neurotransmitter release from a neuron. One example of a modified botulinum toxin is a botulinum toxin that has a light chain from one botulinum toxin serotype (such as serotype A), and a heavy chain from a different botulinum toxin serotype (such as serotype B). Another example of a modified botulinum toxin is a botulinum toxin coupled to a neurotransmitter, such as substance P.

"Peripheral administration" means administration by means of a peripheral location on a mammal. Peripheral administration includes subdermal, intranasal, intramuscular, intradermal, transdermal, and subcutaneous administration.

"Pharmaceutical composition" means a composition comprising an active pharmaceutical ingredient, such as, for example, a clostridial toxin active ingredient such as a botulinum toxin, and at least one additional ingredient, such as, for example, a stabilizer or excipient or the like. A pharmaceutical composition is therefore a formulation which is suitable for diagnostic or therapeutic administration to a subject, such as a human patient. The pharmaceutical composition can be, for example, in a lyophilized or vacuum dried condition, a solution formed after reconstitution of the lyophilized or vacuum dried pharmaceutical composition, or as a solution or solid which does not require reconstitution.

"Pharmacologically acceptable excipient" is synonymous with "pharmacological excipient" or "excipient" and refers to any excipient that has substantially no long term or permanent detrimental effect when administered to mammal and encompasses compounds such as, *e*.*g*., stabilizing agent, a bulking agent, a cryo-protectant, a lyo-protectant, an additive, a vehicle, a carrier, a diluent, or an auxiliary. An excipient generally is mixed with an active ingredient, or permitted to dilute or enclose the active ingredient and can be a solid, semi-solid, or liquid agent. It is also envisioned that a pharmaceutical composition comprising a Clostridial toxin active ingredient can include one or more pharmaceutically acceptable excipients that facilitate processing of an active ingredient into pharmaceutically acceptable compositions. Insofar as any pharmacologically acceptable excipient is not incompatible with the Clostridial toxin active ingredient, its use in pharmaceutically acceptable compositions is contemplated. Non-limiting examples of pharmacologically acceptable excipients can be found in, *e*.*g*., Pharmaceutical Dosage Forms and Drug Delivery Systems (Howard C. Ansel et al., eds., Lippincott Williams & Wilkins Publishers, 7th ed. 1999); Remington: The Science and Practice of Pharmacy (Alfonso R. Gennaro ed., Lippincott, Williams & Wilkins, 20th ed. 2000); Goodman & Gilman's The Pharmacological Basis of Therapeutics (Joel G. Hardman et al., eds., McGraw-Hill Professional, 10th ed. 2001); and Handbook of Pharmaceutical Excipients (Raymond C. Rowe et al., APhA Publications, 4th edition 2003), each of which is hereby incorporated by reference in its entirety.

"Stabilizing agent", "stabilization agent" or "stabilizer" means a substance that acts to stabilize a Clostridial toxin active ingredient.

"Stabilizers" can include excipients, and can include protein and non-protein molecules.

"TEM" as used herein, is synonymous with "Targeted Exocytosis Modulator" or "retargeted endopeptidase." Generally, a TEM comprises an enzymatic domain from a Clostridial toxin light chain, a translocation domain from a Clostridial toxin heavy chain, and a targeting domain. The targeting domain of a TEM provides an altered cell targeting capability that targets the molecule to a receptor other than the native Clostridial toxin receptor utilized by a naturally-occurring Clostridial toxin. This re-targeted capability is achieved by replacing the naturally-occurring binding domain of a Clostridial toxin with a targeting domain having a binding activity for a non-Clostridial toxin receptor. Although binding to a non-Clostridial toxin receptor, a TEM undergoes all the other steps of the intoxication process including internalization of the TEM/receptor complex into the cytoplasm, formation of the pore in the vesicle membrane and di-chain molecule, translocation of the enzymatic domain into the cytoplasm, and exerting a proteolytic effect on a component of the SNARE complex of the target cell.

"Symptoms" related to migraine refers to any and all medically recognized symptoms of migraine. Non-limiting examples of such symptoms include vertigo, nausea, vomiting, fatigue, aura, photophobia, and phonophobia. Symptoms can also include constipation, mood changes (for example from depression to euphoria), food cravings, neck stiffness, increased thirst and urination, frequent yawning, visual phenomena (such as seeing various shapes, bright spots or flashes of light) vision loss, pins and needles sensations in an arm or leg, weakness or numbness in the face or one side of the body, difficulty speaking, hearing noises or music, uncontrollable jerking or other movements, pain on one or both sides of the head, pain that throbs or pulses, sensitivity to light, sound, and sometimes smell and touch, nausea and vomiting,

"Therapeutic formulation" means a formulation can be used to treat and thereby alleviate a disorder or a disease, such as, for example, a headache or headache-associated symptoms.

"Topical administration" excludes systemic administration of the neurotoxin. In other words, and unlike conventional therapeutic transdermal methods, topical administration of botulinum toxin does not result in significant amounts, such as the majority of, the neurotoxin passing into the circulatory system of the patient.

"Treating" means to alleviate (or to eliminate) at least one symptom of a condition or disorder, such as, for example, a headache, or headache-associated symptoms, either temporarily or permanently.

"Variant" means a clostridial neurotoxin, such as wild-type botulinum toxin serotype A, B, C, D, E, F,r G, H, X , En or mosaic botulinum toxins that has been modified by the replacement, modification, addition or deletion of at least one amino acid relative to wild-type botulinum toxin, which is recognized by a target cell, internalized by the target cell, and catalytically cleaves a SNARE (SNAP (Soluble NSF Attachment Protein) Receptor) protein in the target cell.

An example of a variant neurotoxin component can comprise a variant light chain of a botulinum toxin having one or more amino acids substituted, modified, deleted and/or added. This variant light chain may have the same or better ability to prevent exocytosis, for example, the release of neurotransmitter vesicles. Additionally, the biological effect of a variant may be decreased or increased compared to the parent chemical entity. For example, a variant light chain of a botulinum toxin type A having an amino acid sequence removed may have a shorter biological persistence than that of the parent (or native) botulinum toxin type A light chain.

In some embodiments, the clostridial derivative of the present method includes a native, recombinant clostridial toxin, recombinant modified toxin, fragments thereof, targeted exocytosis modulators (TEMs), or combinations thereof. In some embodiments, the clostridial derivative is a botulinum toxin. In alternative embodiments, the clostridial derivative is a TEM.

In some embodiments, the botulinum neurotoxin can be a modified neurotoxin, that is a botulinum neurotoxin which has at least one of its amino acids deleted, modified or replaced, as compared to a native toxin, or the modified botulinum neurotoxin can be a recombinant produced botulinum neurotoxin or a derivative or fragment thereof. In certain embodiments, the modified toxin has an altered cell targeting capability for a neuronal or non-neuronal cell of interest. This altered capability is achieved by replacing the naturally-occurring targeting domain of a botulinum toxin with a targeting domain showing a selective binding activity for a non- botulinum toxin receptor present in a non- botulinum toxin target cell. Such modifications to a targeting domain result in a modified toxin that is able to selectively bind to a non-botulinum toxin receptor (target receptor) present on a non-botulinum toxin target cell (re-targeted). A modified botulinum toxin with a targeting activity for a non-botulinum toxin target cell can bind to a receptor present on the non-botulinum toxin target cell, translocate into the cytoplasm, and exert its proteolytic effect on the SNARE complex of the target cell. In essence, a botulinum toxin light chain comprising an enzymatic domain is intracellularly delivered to any desired cell by selecting the appropriate targeting domain.

In some embodiments, the clostridial derivative is a botulinum toxin, which is selected from the group consisting of botulinum toxin types A, B, C₁, D, E, F,G, H, X, En and mosaic botulinum toxins. In one embodiment, the clostridial derivative of the present method is a botulinum toxin type A. The botulinum toxin can be a recombinant botulinum neurotoxin, such as botulinum toxins produced by *E. coli.*

The clostridial derivative, such as a botulinum toxin, for use according to the present invention can be stored in lyophilized, vacuum dried form in containers under vacuum pressure or as stable liquids. Prior to lyophilization the botulinum toxin can be combined with pharmaceutically acceptable excipients, stabilizers and/or carriers, such as, for example, albumin, or the like. Acceptable excipients or stabilizers include protein excipients, such as albumin or gelatin, or the like, or non- protein excipients, including poloxamers, saccharides, polyethylene glycol, or the like. In embodiments containing albumin, the albumin can be, for example, human serum albumin or recombinant human albumin, or the like. The lyophilized material can be reconstituted with a suitable liquid such as, for example, saline, water, or the like to create a solution or composition containing the botulinum toxin to be administered to the patient.

In some embodiments, to increase the resident time of the clostridial derivative in the joint, the clostridial derivative is provided in a controlled release system comprising a polymeric matrix encapsulating the clostridial derivative, wherein fractional amount of the clostridial derivative is released from the polymeric matrix over a prolonged period of time in a controlled manner. Controlled release neurotoxin systems have been disclosed for example in U.S. patents 6,585,993; 6,585,993; 6,306,423 and 6,312,708, each of which is hereby incorporated by reference in its entirety. The therapeutically effective amount of the clostridial derivative, for example a botulinum toxin, administered according to the present method can vary according to the potency of the toxin and particular characteristics of the condition being treated, including its severity and other various patient variables including size, weight, age, and responsiveness to therapy. The potency of the toxin is expressed as a multiple of the LD₅₀ value for the mouse, one unit (U) of toxin being defined as being the equivalent amount of toxin that kills 50% of a group of 18 to 20 female Swiss-Webster mice, weighing about 20 grams each.

The therapeutically effective amount of the botulinum toxin according to the present method can vary according to the potency of a particular botulinum toxin, as commercially available Botulinum toxin formulations do not have equivalent potency units. For example, one unit of BOTOX^{®} (onabotulinumtoxinA), a botulinum toxin type A available from Allergan, Inc., has a potency unit that is approximately equal to 3 to 5 units of DYSPORT^{®} (abobotulinumtoxinA), also a botulinum toxin type A available from Ipsen Pharmaceuticals. In some embodiments, the amount of abobotulinumtoxinA, (such as DYSPORT^{®}), administered in the present method is about three to four times the amount of onabotulinumtoxinA (such as BOTOX^{®}) administered, as comparative studies have suggested that one unit of onabotulinumtoxinA has a potency that is approximately equal to three to four units of abobotulinumtoxinA. MYOBLOC^{®}, (known as NEUROBLOC^{®} outside the United States) a botulinum toxin type B available from Elan, currently USWorldmeds , has been reported to have a much lower potency unit relative to BOTOX^{®}. In some embodiments, the botulinum neurotoxin can be a pure toxin, devoid of complexing proteins, such as XEOMIN^{®} (incobotulinumtoxinA). The quantity of toxin administered and the frequency of its administration will be at the discretion of the physician responsible for the treatment and will be commensurate with questions of safety and the effects produced by a particular toxin formulation. In some embodiments, the Clostridial derivative is selected from onabotulinumtoxinA, incobotulinumtoxinA, abotulinumtoxinA, daxibotulinumtoxinA, prabotulinumtoxinA, and rimabotulinumtoxinB.

Without wishing to be bound by theory a physiological mechanism can be set forth to explain the efficacy of the peripheral administration of CGRP antagonist and botulinum toxin. Peripheral administration of a combination of a CGRP antagonist and a botulinum toxin in the region of a peripheral nerve according to the methods disclosed herein is believed to permit the CGRP antagonist and botulinum toxin to either be administered to a site in the region of a patient's cranium, neck or shoulder, and/or to reduce afferent, sensory input from a site in the region of the patient's cranium, neck or shoulder, to thereby influence intracranial neurons involved in a neurological disorder, including headache, migraine and related symptoms. In addition, the combination allows for lower doses of both and/or each component. This results in decreased side effects. Furthermore, efficacy is increased by having a multimodal mechanism of action from the combination of therapeutic agents. CGRP is only one of many neuro-transmitters released by the peripheral nerves and botulinum toxins have the ability to block more than CGRP release. CGRP antagonists will have enhanced action on neurological diseases, including headache, migraine and related symptoms, by combination with botulinum toxins as these will block other neurotransmitters such as substance P and glutamate.

Administration in the region of a peripheral nerve, a cranial nerve, or combination therof, including but not limited to a trigeminal, occipital, autonomic, spinal or cervical sensory nerve(s) of a CGRP antagonist in combination with a botulinum toxin in accordance with the present disclosure can also block progression of neurological disorders, including headache, migraine and related symptoms mediated by repeated sensory input to the cortex from a sensory nerve and also from autonomic nervous system components.

Methods and medicaments for treating, preventing, alleviating or reducing the frequency of occurrence of migraine, headaches, and related symptoms according to the present disclosure can comprise a CGRP antagonist in combination with a clostridial derivative, for example, a botulinum toxin, for peripherally administration in the region of a peripheral nerve of a patient. The CGRP antagonist is administered in a therapeutically effective amount to alleviate at least one symptom of a neurological disorder, including headache, migraine and related symptoms.

Non-limiting examples of centrally mediated disorders include migraine, epilepsy, chronic pain (such as central sensitization chronic pain, central post stroke pain, regional pain, phantom limb pain, or demyelinating disease pain), reflex sympathetic dystrophy, allodynic states; chronic neurological conditions in which kindling is part of the disease process; mood disorders (including bipolar disease) and movement; muscle-related and neuromuscular disorders.

In some embodiments, the disclosure provides for the peripheral administration in the region of a peripheral nerve, of a combination of CGRP antagonists and optionally a clostridial derivative, for example a botulinum toxin, to treat (including alleviate and/or prevent) a variety of neurological disorders, including headache, migraine and related symptoms. The peripheral administration in the region of a peripheral nerve, a cranial nerve, a ganglion or combinations thereof of a combination of a CGRP antagonist and optionally a botulinum toxin can provide significant and long-lasting relief from a variety of neurological disorders including headache, migraine and related symptoms.

In some embodiments, the clostridial derivative, for example a botulinum toxin, is administered to a trigeminal nerve. Trigeminal nerve administration of botulinum toxins has been disclosed for example in U.S. Patents Nos. 8,609,112; 8,609,113; 8,734,810; 8,717,572; 9,238,061 and 10,064,921; each of which is hereby incorporated by reference in its entirety.

In some embodiments, the clostridial derivative, for example a botulinum toxin, is administered to a suture line. Suture line administration of botulinum toxins has been disclosed for example in U.S. Patents Nos. 8,617,571; 9,248,168; 9,827,297; and 10,220,079; each of which is hereby incorporated by reference in its entirety.

### EXAMPLES

The following non-limiting examples provide those of ordinary skill in the art with possible case scenarios and specific methods to treat conditions within the scope of the present disclosure and are not intended to limit the scope of the disclosure. In the following examples administration of a CGRP antagonist in combination with a botulinum toxin can be carried out. For example, by topical application (cream or transdermal patch), subcutaneous injection, or subdermal implantation of a controlled release implant.

### Example 1

A 43-year-old female that had a long history of migraine dating back to her early teens was transformed to chronic migraine over 10 years ago. The patient saw many headache experts and tried numerous oral preventive medications including topiramate without headache control. The patient was treated with OnabotulinumtoxinA over a period of 4 years using the PHASE III Research Evaluating Migraine Prophylaxis Therapy (PREEMPT) protocol with both fixed sites and following the pain sites of injections with a dose of 155 to 195 units every 12 weeks. The patient's headache days reduced from near daily to about half the days in the month on this treatment, an about 50% improvement from her baseline. The patient under the OnabotulinumtoxinA treatment reduced migraine days from near daily (about 28 days per month) to 15 days per month. Having received OnabotulinumtoxinA for many years, the patient's response was very predictable with each treatment. The patient subsequently received subcutaneous injection of CGRP monoclonal antibody erenumab subcutaneously at a dose of 140mg on a monthly basis. Using the combination treatment for over 6 months the patient documented a significant further improvement of her headache frequency. After six months of combination therapy treatment with erenumab and OnabotulinumtoxinA the number of headache days per month was reduced to 3 days. This response far exceeds the expected response of erenumab alone.

### Example 2

A 40-year-old female that has a long history of migraine is treated with OnabotulinumtoxinA using the PHASE III Research Evaluating Migraine Prophylaxis Therapy (PREEMPT) protocol with both fixed sites and following the pain sites of injections with a dose of 155 to 195 units every 12 weeks. The patient is concurrently undergoing treatment with 50 mg of ubrogepant as needed for breakthrough migraine attacks. Using the combination treatment for over six months the frequency of the patient's migraine attack and headache intensity can be reduced.

### Example 3

A 43-year-old female has a long history of migraine. She transformed from episodic to chronic migraine and has an average of 15 headache days a month. She is treated with OnabotulinumtoxinA using the PREEMPT protocol with both fixed sites and following the pain sites of injections with a dose of 155 to 195 units every 12 weeks. She continues with this treatment for three years. Her headache frequency stabilizes at 10 days a month. She then discontinues OnabotulinumtoxinA treatment. She remains off of treatment for four months. Her headache increases to 12 to 14 days per month. She is stable at this level. She then starts treatment with atogepant at a dose of 60 mg b.i. d. Her headaches reduced to 9 days a month. She is stable at this level for six months. She continues on atogepant and OnabotulinumtoxinA treatment is reinstituted. After six months of continuous atogepant treatment and OnabotulinumtoxinA treatment every three months, her headache frequency is reduced to two days a month.

### Example 4

A 60-year-old male has a long history of chronic migraine is treated with OnabotulinumtoxinA using the PREEMPT protocol with both fixed sites and following the pain sites of injections with a dose of 155 to 195 units every 12 weeks. His baseline frequency is 22 days/month, and post treatment, the frequency is 14 days/month. He continues with this treatment for three years with this treatment profile. He then discontinues OnabotulinumtoxinA treatment, and remains off of treatment for four months. He begins treatment with ererumab at 140 mg/month for 6 months. His headache frequency is decreased from 22 days/month to 10 days/month. However, he develops severe constipation that required weekly enemas. After 4 months of treatment, the dose of erenumab is decreased to 70 mg/ month and the constipation resolved. However, the headache frequency stabilizes at 15 days/month. After 3 months, the PREEMPT OnabotulinumtoxinA protocol is reinstituted every 3 months while he remains on erenumab is continued at 70mg/month. The headache frequency decreases to 5 days/month. He continues on the therapy and there is no constipation.

### Example 5

A 42-year-old female was presented with a history of migraine dating to her teens. She had near daily escalating headache by her thirties. She relied on analgesics, mainly on Fiorinal (Butalbital, Aspirin, and Caffeine Capsules, USP) to control her headaches. She used Fiorinal nearly every day. She also tried Elavil (amitriptyline) and Topamax (topiramate) with minimal change. She had been treated with onabotulinumtoxinA using the PREEMPT protocol for seven years. Using the onabotulinumtoxinA treatment, her headaches were reduced from 30/30 days to 10/30 with an intensity of 6-7 out of a scale of 10. Namenda (memantine) treatment was added with minimal benefit. Treatment with erenumab (140 mg/month) was started and continued for 12 months. The onabotulinumtoxinA treatment continued using the PREEMPT protocol. The patient exhibited no side effects from the combined treatments. The frequency and intensity of headaches progressively improved over the 12-month period of combination therapy. After 12 months the patient averaged frequency of headaches of 5/30 days with an average intensity of 4 on a scale of 10. Prior to the beginning of the combination therapy, the patient would have had at least two emergency room visits per year. During the 12-month period of combination therapy, the patient had no emergency room visits.

### Example 6

A 59-year-old woman was presented with a long history of migraines. In her teens the episodes were once a month just before her menses. As she got older her headache frequency increased. She reached 15 headache days a month by her late 30's. She had at least 8 of these days which met criteria for migraine without aura, and responded to triptans.

She tried many different oral preventive medications without any benefit. Topiramate caused cognitive slowing, Amitriptyline caused weight gain, Propranolol limited her exercise tolerance; and in addition, none of these reduced her headache frequency. As a result, she was started on BOTOX^{®} (onabotulinumtoxinA). Her first cycle was 155 units in the standard 31 PREEMPT sites. She had only a slight reduction in headache intensity from 9 out of 10 to 7 out of 10 and thus her dose was increased to 195 units. Her headaches reduced from 15-20 days a month to 10-12 days a month. She completed 5 courses of treatment, but due to lack of further improvement decided to discontinue BOTOX^{®} treatment. She went 6 months without any preventive medications and averaged 15-20 headache days a month during this time. Due to significant migraine disability she tried a CGRP monoclonal antibody. She received Ajovy (fremanezumab) 675 mg as three 225 mg subcutaneous injections every 3 months for 6 months and again her headaches reduced to 10-12 days a month with lower intensity. In discussion with her the Botox was added back at 195 units. The sequence of treatment was subcutaneous administration of Ajovy at 675 mg and 6 weeks later Botox injections at 195 units with follow the pain sites in both temporalis and occipitalis muscles. Over the next 2 months with this combination approach her headaches reduced to one day a week, with intensity of 4-5 out of 10.

### STATEMENTS

1. A method for treating, preventing, alleviating or reducing the frequency of occurrence of headache in a patient in need thereof, comprising administering to the patient an antagonist of calcitonin gene-related peptide (CGRP-antagonist), wherein said patient is concurrently undergoing treatment with a clostridial derivative.
2. A method for treating, preventing, alleviating or reducing the frequency of occurrence of headache in a patient in need thereof, comprising administering to the patient;
   (a) an antagonist of calcitonin gene-related peptide (CGRP-antagonist); and,
   (b) a clostridial derivative.
3. A method for treating, preventing, alleviating or reducing the frequency of occurrence of migraine or a symptom of migraine in a patient in need thereof, comprising administering to the patient an antagonist of calcitonin gene-related peptide (CGRP-antagonist), wherein said patient is concurrently undergoing treatment with a clostridial derivative.
4. A method for treating, preventing, alleviating or reducing the frequency of occurrence of migraine or a symptom of migraine in a patient in need thereof, comprising administering to the patient;
   (a) an antagonist of calcitonin gene-related peptide (CGRP-antagonist); and,
   (b) a clostridial derivative.
5. The method according to statements 3 or 4 wherein the headache is a migraine headache, a cluster headache, a hemicrania continua headache, a chronic headache, a tension headache, a chronic tension headache, or any combination thereof.
6. The method according to any of statements 1-4 wherein said clostridial derivative is a botulinum toxin of immunotype A, B, C, D, E, F, G, H, X, or En.
7. The method according to statement 6 wherein the botulinum toxin is onabotulinumtoxinA.
8. The method according to any of the above statements wherein said CGRP-antagonist is an anti-calcitonin gene-related peptide receptor antibody (anti-CGRP antibody) or antigen-binding fragment thereof.
9. The method according to statement 8 wherein said antibody is selected from galcanezumab, fremanezumab, eptinezumab, and erenumab.
10. The method according to statement 9 wherein said antibody is erenumab.
11. The method according to any of the above statements wherein said antagonist of CGRP receptor is selected from ubrogepant, atogepant, rimegepant or a pharmaceutically acceptable salt thereof.
12. The method according to any of statements 3-11 wherein said patient experiences reduced frequency of one or more symptoms or side effects of migraine selected from sinusitis, nausea, nasopharyngitis in comparison with a patient undergoing treatment with CGRP-antagonist without botulinum toxin treatment.
13. The method according to any of statements 3-12 wherein the frequency of symptoms associated with one or more stages of migraine selected from prodrome, aura, headache or postdrome is reduced.
14. The method according to statement 13 wherein one or more frequency of symptoms associated with prodrome stage of migraine selected from photophobia, appetite changes, cognition and concentration difficulties, cold extremities, diarrhea or other bowel changes, excitement or irritability, fatigue, frequent urination, memory changes, weakness, yawning and stretching is reduced.
15. The method according to statement 13 wherein one or more frequency of symptoms associated with aura stage of migraine selected from seeing bright spots or flashes of light, vision loss, seeing dark spots, tingling sensations, speech problems, aphasia, and tinnitus is reduced.
16. The method according to statement 13 wherein one or more frequency of symptoms associated with attack stage of migraine selected from photophobia, gastric stasis, pulsating or throbbing pain on one or both sides of the head, extreme sensitivity to light, sounds, or smells, worsening pain during physical activity, nausea and vomiting, abdominal pain or heartburn, loss of appetite, lightheadedness, blurred vision, and fainting is reduced.
17. The method according to any of statements 3-13 wherein said migraine is migraine without aura stage.
18. The method according to any of the about statements wherein said migraine is menstrual migraine, familial hemiplegic migraine or chronic migraine.
19. The method according to any of the above statements wherein said anti-CGRP antibody or fragment thereof is administered at a dosage that is about 20% or 30% or 40% or 50% or 60% or 70% or 80% lower than the recommended dosage for the anti-CGRP antibody monotherapy.
20. The method according to any of the above statements wherein said clostridial derivative is administered once every four weeks, five weeks, six weeks, seven weeks, eight weeks, nine weeks, ten weeks, eleven weeks, twelve weeks, thirteen weeks, fourteen weeks, fifteen weeks, sixteen weeks, seventeen weeks, eighteen weeks, nineteen weeks, twenty weeks, twenty one weeks, twenty two weeks, twenty three weeks, twenty four weeks, twenty five weeks, twenty six weeks or more.
21. The method according to any of the above statements wherein said clostridial derivative is administered to a peripheral nerve, a cranial nerve, or combinations thereof.
22. The method according to any of statements 10-21 wherein said anti-calcitonin gene-related peptide receptor antibody or antigen-binding fragment thereof is administered to a peripheral nerve, a cranial nerve, a spinal nerve or combinations thereof.
23. The method according to any of the above statements wherein said patient is administered one or more additional medications for the treatment of headache.
24. The method according to statement 23 wherein said additional medication is selected from opioid analgesics, Cox-2 inhibitors, barbiturates, sodium channel modulators, potassium channel modulators, calcium channel blockers, local anesthetics, monoamine oxidase inhibitors, leukotriene LTD4 receptor blocker, substance P antagonists, 5-HT3 antagonists and NMDA antagonists.
25. The method according to statement 24 wherein said one or more additional medications is selected from aspirin, ibuprofen, naproxen, acetaminophen, diclofenac, flurbiprofen, meclofenamate, isometheptene, indomethacin; codeine, morphine, hydrocodone, acetyldihydrocodeine, oxycodone, oxymorphone, papaverine, fentanyl, alfentanil, sufentanil, remifentanyl, tramadol, prochlorperazine, celecoxib, rofecoxib, meloxicam, piroxicam, JTE-522, L-745,337, NS398, deracoxib, valdecoxib, iumiracoxib, etoricoxib, parecoxib, 4-(4-cyclohexyl-2-methyloxazol-5-yl)-2 fluorobenzenesulfonamide, (2-(3,5-difluorophenyl)-3 -(4-(methylsulfonyl)phenyl)-2 cyclopenten-1-one, N-[2-(cyclohexyloxy)-4-nitrophenyl]methanesulfonamide, 2-(3,4 difluorophenyl)-4-(3-hydroxy-3-methylbutoxy)-5-[4-(methylsulfonyl) phenyl]-3(2H) pyridazinone, 2-[(2,4-dichloro-6-methylphenyl) amino]-5-ethyl-benzeneacetic acid, (3Z) 3-[(4-chlorophenyl) [4-(methylsulfonyl)phenyl] methylene]dihydro-2(3H)-furanone, (S)-6,8-dichloro-2-(trifluoromethyl)-2H-1-benzopyran-3-carboxylic acid, amobarbital, butalbital, cyclobarbital, pentobarbital, allobarbital, methylphenobarbital, phenobarbital, secobarbital, vinylbital, verapamil, ciltiazem, Nifedipine, lidocaine, tetracaine, prilocaine, bupivicaine, mepivacaine, etidocaine, procaine, benzocaine, phehelzine, isocarboxazid, dichloralphenazone, nimopidine, metoclopramide, capsaicin receptor agonists, captopril, tiospirone, a steroid, caffeine, metoclopramide, domperidone, scopolamine, dimenhydrinate, diphenhydramine, hydroxyzine, diazepam, lorazepam, chlorpromazine, methotrimeprazine, perphenazine, prochlorperazine, promethazine, trifluoperazine, triflupromazine, benzquinamide, bismuth subsalicylate, buclizine, cinnarizine, cyclizine, diphenidol, dolasetron, domperidone, dronabinol, droperidol, haloperidol, metoclopramide, nabilone, thiethylperazine, trimethobenzemide, and eziopitant, Meclizine, domperidone, ondansetron, tropisetron granisetron dolasetron, hydrodolasetron, palonosetron, alosetron, cilansetron, cisapride, renzapride metoclopramide, galanolactone, phencyclidine, ketamine, dextromethorphan, and isomers, pharmaceutically acceptable salts, esters, conjugates, or prodrugs thereof.
26. The method according to any of statements 9-25 wherein said anti-CGRP antibody is erenumab.
27. The method according to statement 26 wherein erenumab is administered subcutaneously at a dose of about 5 mg to about 500 mg every one, two, three, four, five, six, seven, eight, nine or ten weeks.
28. The method according to statement 26 wherein erenumab is administered subcutaneously at a dose of about 10 mg to about 200 mg every one, two, three, four, five, six, seven, eight, nine or ten weeks.
29. The method according to statement 26 wherein erenumab is administered subcutaneously at a dose of about 25 mg to about 150 mg every one, two, three, four, five, six, seven, eight, nine or ten weeks.
30. The method according to statement 26 wherein erenumab is administered subcutaneously at a dose of about 90 mg to about 120 mg every one, two, three, four, five, six, seven, eight, nine or ten weeks.
31. The method according to statement 26 wherein erenumab is administered subcutaneously at a dose of about 50 mg to about 60 mg every one, two, three, four, five, six, seven, eight, nine or ten weeks.
32. The method according to statement 26 wherein erenumab is administered subcutaneously at a dose of about 70 mg every one, two, three, four, five, six, seven, eight, nine or ten weeks.
33. The method according to statement 26 wherein erenumab is administered subcutaneously at a dose of about 140 mg every one, two, three, four, five, six, seven, eight, nine or ten weeks.
34. The method according to statement 26 wherein erenumab is administered subcutaneously at a monthly dose of about 140 mg.
35. The method according to statement 26 wherein erenumab is administered subcutaneously at a monthly dose of about 70 mg.
36. The method according to statement 26 wherein erenumab is administered subcutaneously at a dose of about 140 mg every two months.
37. The method according to statement 26 wherein erenumab is administered subcutaneously at a dose of about 70 mg every two months.
38. The method according to statement 26 wherein erenumab is administered subcutaneously at a dose of about 140 mg every three months.
39. The method according to statement 26 wherein erenumab is administered subcutaneously at a dose of about 70 mg every three months.
40. The method according to any of statements 9-25 wherein said anti-CGRP antibody is galcanezumab.
41. The method according to statement 40 wherein galcanezumab is administered subcutaneously at a dose of about 10 mg to about 500 mg every one, two, three, four, five, six, seven, eight, nine or ten weeks.
42. The method according to statement 40 wherein galcanezumab is administered subcutaneously at a dose of about 50 mg to about 300 mg every one, two, three, four, five, six, seven, eight, nine or ten weeks.
43. The method according to statement 40 wherein galcanezumab is administered subcutaneously at a dose of about 75 mg to about 250 mg every one, two, three, four, five, six, seven, eight, nine or ten weeks.
44. The method according to statement 40wherein galcanezumab is administered subcutaneously at a dose of about 75 mg to about 100 mg every one, two, three, four, five, six, seven, eight, nine or ten weeks.
45. The method according to statement 40 wherein galcanezumab is administered subcutaneously at a dose of about 150 mg to about 220 mg every one, two, three, four, five, six, seven, eight, nine or ten weeks.
46. The method according to statement 40 wherein galcanezumab is administered subcutaneously at a dose of about 120 mg every one, two, three, four, five, six, seven, eight, nine or ten weeks.
47. The method according to statement 40 wherein galcanezumab is administered subcutaneously at a dose of about 240 mg every one, two, three, four, five, six, seven, eight, nine or ten weeks.
48. The method according to statement 40 wherein galcanezumab is administered subcutaneously at a monthly dose of about 240 mg.
49. The method according to statement 40 wherein galcanezumab is administered subcutaneously at a monthly dose of about 120 mg.
50. The method according to statement 40 wherein galcanezumab is administered subcutaneously at a dose of about 240 mg every two months.
51. The method according to statement 40 wherein galcanezumab is administered subcutaneously at a dose of about 120 mg every two months.
52. The method according to statement 40 wherein galcanezumab is administered subcutaneously at a dose of about 240 mg every three months.
53. The method according to statement 40 wherein galcanezumab is administered subcutaneously at a dose of about 120 mg every three months.
54. The method according to any of statements 9-25 wherein said anti-CGRP antibody is fremanezumab.
55. The method according to statement 54 wherein fremanezumab is administered subcutaneously at a dose of about 100 mg to about 1000 mg every one, two, three, four, five, six, seven, eight, nine or ten weeks.
56. The method according to statement 54 wherein fremanezumab is administered subcutaneously at a dose of about 150 mg to about 700 mg every one, two, three, four, five, six, seven, eight, nine or ten weeks.
57. The method according to statement 54 wherein fremanezumab is administered subcutaneously at a dose of about 150 mg to about 500 mg every one, two, three, four, five, six, seven, eight, nine or ten weeks.
58. The method according to statement 54 wherein fremanezumab is administered subcutaneously at a dose of about 150 mg to about 200 mg every one, two, three, four, five, six, seven, eight, nine or ten weeks.
59. The method according to statement 54 wherein fremanezumab is administered subcutaneously at a dose of about 150 mg to about 500 mg every one, two, three, four, five, six, seven, eight, nine or ten weeks.
60. The method according to statement 54 wherein fremanezumab is administered subcutaneously at a dose of about 225 mg every one, two, three, four, five, six, seven, eight, nine or ten weeks.
61. The method according to statement 54 wherein fremanezumab is administered subcutaneously at a dose of about 450 mg every one, two, three, four, five, six, seven, eight, nine or ten weeks.
62. The method according to statement 54 wherein fremanezumab is administered subcutaneously at a dose of about 675 mg every one, two, three, four, five, six, seven, eight, nine or ten weeks.
63. The method according to statement 54 wherein fremanezumab is administered subcutaneously at a monthly dose of about 225 mg.
64. The method according to statement 54 wherein fremanezumab is administered subcutaneously at a monthly dose of about 450 mg.
65. The method according to statement 54 wherein fremanezumab is administered subcutaneously at a monthly dose of about 675 mg.
66. The method according to statement 54 wherein fremanezumab is administered subcutaneously at a dose of about 225 mg every two months.
67. The method according to statement 54 wherein fremanezumab is administered subcutaneously at a dose of about 450 mg every two months.
68. The method according to statement 54 wherein fremanezumab is administered subcutaneously at a dose of about 225 mg every three months.
69. The method according to statement 54 wherein fremanezumab is administered subcutaneously at a dose of about 450 mg every three months.
70. The method according to statement 54 wherein fremanezumab is administered subcutaneously at a dose of about 675 mg every three months.
71. The method according to any of statements 9-25 wherein said anti-CGRP antibody is eptinezumab.
72. The method according to statement 71 wherein eptinezumab is administered subcutaneously at a dose of about 50 mg to about 1000 mg every one, two, three, four, five, six, seven, eight, nine or ten weeks.
73. The method according to statement 71 wherein eptinezumab is administered subcutaneously at a dose of about 100 mg to about 710 mg every one, two, three, four, five, six, seven, eight, nine or ten weeks.
74. The method according to statement 71 wherein eptinezumab is administered subcutaneously at a dose of about 200 mg to about 500 mg every one, two, three, four, five, six, seven, eight, nine or ten weeks.
75. The method according to statement 71 wherein eptinezumab is administered subcutaneously at a dose of about 250 mg to about 350 mg every one, two, three, four, five, six, seven, eight, nine or ten weeks.
76. The method according to statement 71 wherein eptinezumab is administered subcutaneously at a dose of about 300 mg every one, two, three, four, five, six, seven, eight, nine or ten weeks.
77. The method according to statement 71 wherein eptinezumab is administered subcutaneously at a monthly dose of about 100 mg.
78. The method according to statement 71 wherein eptinezumab is administered subcutaneously at a monthly dose of about 200 mg.
79. The method according to statement 71 wherein eptinezumab is administered subcutaneously at a monthly dose of about 300 mg.
80. The method according to statement 71 wherein eptinezumab is administered subcutaneously at a dose of about 100 mg every two months.
81. The method according to statement 71 wherein eptinezumab is administered subcutaneously at a dose of about 200 mg every two months.
82. The method according to statement 71 wherein eptinezumab is administered subcutaneously at a dose of about 300 mg every two months.
83. The method according to statement 71 wherein eptinezumab is administered subcutaneously at a dose of about 100 mg every three months.
84. The method according to statement 71 wherein eptinezumab is administered subcutaneously at a dose of about 200 mg every three months.
85. The method according to statement 71 wherein eptinezumab is administered subcutaneously at a dose of about 300 mg every three months.
86. The method according to any of statements 9-25 wherein said headache is migraine headache and said patient suffers from reduced frequency of migraine attacks in comparison to monotherapy treatment with anti-CGRP antibody or CGRP antagonist.
87. The method according to statement 86 wherein said frequency of migraine attacks is reduced by 10%, 20%, 30%, 40%, 50%, 60%, 70%, 80% or 90% in comparison to monotherapy treatment with anti-CGRP antibody or CGRP antagonist.
88. The method according to any of statements 9-25 wherein the number of days said patient experiences migraine attacks per month is reduced in comparison to monotherapy treatment with anti-CGRP antibody or CGRP antagonist.
89. The method according to statement 88 wherein said number of days of migraine attack is reduced by 10%, 20%, 30%, 40%, 50%, 60%, 70%, 80% or 90% in comparison to monotherapy treatment with anti-CGRP antibody or CGRP antagonist.
90. The method according to any of the above statements wherein said patient experiences at least 2 days per month with headache lasting 2, 3, or 4 hours a day or longer.
91. The method according to any of the above statements wherein said patient experiences at least 5 days per month with headache lasting 2, 3, or 4 hours a day or longer.
92. The method according to any of the above statements wherein said patient experiences at least 10 days per month with headache lasting 2, 3, or 4 hours a day or longer.
93. The method according to any of the above statements wherein said patient experiences at least 15 days per month with headache lasting 2, 3, or 4 hours a day or longer.
94. The method according to any of the above statements wherein said patient experiences at least 15 days per month with headache lasting 4 hours a day or longer.
95. The method according to any of statements 90-94 wherein said migraine is chronic migraine.
96. The method according to any of statements 90-94 wherein said migraine is episodic migraine.
97. The method according to any of statements 7-96 wherein said onabotulinumtoxinA is administered at a dose of about 1 unit, about 2 units, about 3 units, about 4 units, about 5 units, about 6 units, about 7 units, about 8 units, about 9 units or about 10 units every one, two, three, four, five, six, seven, eight, nine, ten, eleven, twelve, thirteen, fourteen, fifteen, sixteen weeks, seventeen weeks, eighteen weeks, nineteen weeks, twenty weeks, twenty one weeks, twenty two weeks, twenty three weeks, twenty four weeks, twenty five weeks, twenty six weeks or more.
98. The method according to any of statements 7-96, wherein said onabotulinumtoxinA is administered at a dose of about 10 unit, about 15 units, about 20 units, about 25 units, about 30 units, about 40 units, about 45 units, about 50 units, about 55 units or about 60 units every one, two, three, four, five, six, seven, eight, nine, ten, eleven, twelve, thirteen, fourteen, fifteen or sixteen weeks.
99. The method according to any of any of statements 7-96, wherein said onabotulinumtoxinA is administered at a dose of about 25 unit, about 50 units, about 75 units, about 100 units, about 125 units, about 150 units, about 175 units, about 200 units, about 225 units or about 250 units every one, two, three, four, five, six, seven, eight, nine, ten, eleven, twelve, thirteen, fourteen, fifteen or sixteen weeks.
100. The method according to any of statements 7-96, wherein said onabotulinumtoxinA is administered at a dose of about 1 to about 1,000 units every one, two, three, four, five, six, seven, eight, nine, ten, eleven, twelve, thirteen, fourteen, fifteen or sixteen weeks.
101. The method according to any of statements 7-96, wherein said onabotulinumtoxinA is administered at a dose of about 1 to about 100 units every one, two, three, four, five, six, seven, eight, nine, ten, eleven, twelve, thirteen, fourteen, fifteen or sixteen weeks.
102. The method according to any of statements 7-96, wherein said onabotulinumtoxinA is administered at a dose of about 10 to about 50 units every one, two, three, four, five, six, seven, eight, nine, ten, eleven, twelve, thirteen, fourteen, fifteen or sixteen weeks.
103. The method according to any of statements 97-102 wherein said onabotulinumtoxinA is administered peripherally.
104. The method according to any of statements 97-102 wherein said onabotulinumtoxinA is administered subcutaneously or intramuscularly.
105. The method according to any of statements 97-102 wherein said onabotulinumtoxinA is administered subcutaneously once every month or two months.
106. The method according to any of statements 7-97 or 100-104 wherein said onabotulinumtoxinA is administered at a dose of about 155 units.
107. The method according to any of statements 97-102 wherein said onabotulinumtoxinA is administered to a suture line.
108. The method according to any of the above statements wherein said CGRP-antagonist a) bind to CGRP or CGRP-R and the binding results in a reduction or inhibition of CGRP activity; (b) block CGRP from binding to its receptor(s); (c) block or decrease CGRP receptor activation; (d) inhibit CGRP biological activity or downstream pathways mediated by CGRP signaling function; (e) increase clearance of CGRP; or (f) inhibit or reduce CGRP synthesis, production or release.
109. An improved method for treating, preventing, alleviating or reducing the frequency of occurrence of headache in a patient in need thereof, wherein the improvement comprises administering to the patient an antagonist of calcitonin gene-related peptide(CGRP-antagonist), wherein said patient is concurrently undergoing treatment with a clostridial derivative.
110. An improved method for treating, preventing, alleviating or reducing the frequency of occurrence of headache in a patient in need thereof, the improvement comprises administering to the patient;
   (a) an antagonist of calcitonin gene-related peptide(CGRP-antagonist); and,
   (b) a clostridial derivative.
111. An improved method for treating, preventing, alleviating or reducing the frequency of occurrence of migraine or a symptom of migraine in a patient in need thereof, the improvement comprises administering to the patient an antagonist of calcitonin gene-related peptide (CGRP-antagonist), wherein said patient is concurrently undergoing treatment with a clostridial derivative.
112. An improved method for treating, preventing, alleviating or reducing the frequency of occurrence of migraine or a symptom of migraine in a patient in need thereof, the improvement comprises administering to the patient;
   (a) an antagonist of calcitonin gene-related peptide (CGRP-antagonist); and,
   (b) a clostridial derivative.
113. The method according to statement 109 or 110 wherein the headache is a migraine headache, a cluster headache, a hemicrania continua headache, a chronic headache, a tension headache, a chronic tension headache, a post traumatic headache, a post-ictal headache, or any combination thereof.
114. The method according to any of statements 108-113 wherein said clostridial derivative is a botulinum toxin of serotype A, B, C, D, E, F, G, H, X, or En.
115. The method according to statement 114 wherein the botulinum toxin is onabotulinumtoxinA.
116. The method according to any of statements 109-115 wherein said CGRP-antagonist is an anti-calcitonin gene-related peptide receptor antibody (anti-CGRP antibody) or antigen-binding fragment thereof.
117. The method according to statement 116 wherein said antibody is selected from galcanezumab, fremanezumab, eptinezumab, and erenumab.
118. The method according to any of statements 109-117 wherein said antagonist of CGRP receptor is selected from ubrogepant, atogepant, rimegepant or a pharmaceutically acceptable salt thereof.
119. The method according to any of statements 1-26 or 86-118 wherein said antagonist of CGRP receptor is administered parenterally, preferably intravenously.
120. The method according to statement 119 wherein said antagonist of CGRP receptor is ubrogepant or a pharmaceutically acceptable salt thereof, and ubrogepant is administered at a dose of about 5 to about 500 mg per day.
121. The method according to statement 120 wherein said dose is about 5, 10, 15, 20, 25, 30, 35, 40, 45, 50, 55, 60, 65, 70, 75, 80, 85, 90, 95, 100, 110, 120, 125, 130, 140, 150, 160, 170, 175, 180, 190 or 200 mg per day.
122. The method according to statement 119 wherein said antagonist of CGRP receptor is atogepant or a pharmaceutically acceptable salt thereof, and atogepant is administered at a dose of about 5 to about 500 mg per day.
123. The method according to statement 122 wherein said dose is about 5, 10, 15, 20, 25, 30, 35, 40, 45, 50, 55, 60, 65, 70, 75, 80, 85, 90, 95, 100, 110, 120, 125, 130, 140, 150, 160, 170, 175, 180, 190 or 200 mg per day.
124. The method according to statement 118 wherein said antagonist of CGRP receptor is administered intravenously.
125. The method according to any of the above statements wherein one or more symptoms of migraine is reduced.
126. The method according to statement 124 wherein said symptom is associated with prodrome stage of migraine wherein said symptom is selected from photophobia, appetite changes, cognition and concentration difficulties, cold extremities, diarrhea or other bowel changes, excitement or irritability, fatigue, frequent urination, memory changes, weakness, yawning and stretching.
127. The method according to statement 124 wherein said symptom is associated with aura stage of migraine wherein said symptom is selected from seeing bright spots or flashes of light, vision loss, seeing dark spots, tingling sensations, speech problems, aphasia, and tinnitus.
128. The method according to statement 124 wherein said symptom is associated with attack stage of migraine wherein said symptom is selected from photophobia, gastric stasis, pulsating or throbbing pain on one or both sides of the head, extreme sensitivity to light, sounds, or smells, worsening pain during physical activity, nausea and vomiting, abdominal pain or heartburn, loss of appetite, lightheadedness, blurred vision, and fainting.

## Claims

1. An antagonist of calcitonin gene-related peptide (CGRP-antagonist) for use in a method for treating, preventing, alleviating or reducing the frequency of occurrence of migraine or a symptom of migraine in a patient in need thereof, comprising administering to the patient the CGRP-antagonist, wherein said patient is concurrently undergoing treatment with a clostridial derivative.

2. The CGRP-antagonist for use according to claim 1, wherein the headache is a migraine headache, a cluster headache, a hemicrania continua headache, a chronic headache, a tension headache, a chronic tension headache, or any combination thereof.

3. The CGRP-antagonist for use according to claims 1 or 2, wherein said CGRP-antagonist is an anti-calcitonin gene-related peptide receptor antibody (anti-CGRP antibody) or antigen-binding fragment thereof.

4. The CGRP-antagonist for use according to claim 3, wherein said antibody is selected from galcanezumab, fremanezumab, eptinezumab, and erenumab.

5. The CGRP-antagonist for use according to claim 4, wherein said antibody is erenumab.

6. The CGRP-antagonist for use according to any one of claims 1-4, wherein said antagonist of CGRP receptor is selected from ubrogepant, atogepant, rimegepant or a pharmaceutically acceptable salt thereof.

7. The CGRP-antagonist for use according to any one of claims 1-6, wherein said patient experiences reduced frequency of one or more symptoms or side effects of migraine selected from sinusitis, nausea, nasopharyngitis in comparison with a patient undergoing treatment with CGRP-antagonist without botulinum toxin treatment.

8. The CGRP-antagonist for use according to any one of claims 1-7, wherein the frequency of symptoms associated with one or more stages of migraine selected from prodrome, aura, headache or postdrome is reduced.

9. The CGRP-antagonist for use according to claim 8, wherein one or more frequency of symptoms associated with prodrome stage of migraine selected from photophobia, appetite changes, cognition and concentration difficulties, cold extremities, diarrhea or other bowel changes, excitement or irritability, fatigue, frequent urination, memory changes, weakness, yawning and stretching is reduced.

10. The CGRP-antagonist for use according to claim 8, wherein one or more frequency of symptoms associated with aura stage of migraine selected from seeing bright spots or flashes of light, vision loss, seeing dark spots, tingling sensations, speech problems, aphasia, and tinnitus is reduced.

11. The CGRP-antagonist for use according to claim 8, wherein one or more frequency of symptoms associated with attack stage of migraine selected from photophobia, gastric stasis, pulsating or throbbing pain on one or both sides of the head, extreme sensitivity to light, sounds, or smells, worsening pain during physical activity, nausea and vomiting, abdominal pain or heartburn, loss of appetite, lightheadedness, blurred vision, and fainting is reduced.

12. The CGRP-antagonist for use according to any one of claims 1-8, wherein said migraine is migraine without aura stage.

13. The CGRP-antagonist for use according to any one of claims 1-12, wherein said migraine is menstrual migraine, familial hemiplegic migraine or chronic migraine.

14. The CGRP-antagonist for use according to any one of claims 1-13, wherein said anti-CGRP antibody or fragment thereof is administered at a dosage that is about 20% or 30% or 40% or 50% or 60% or 70% or 80% lower than the recommended dosage for the anti-CGRP antibody monotherapy.

15. The CGRP-antagonist for use according to any one of claims 1-14, wherein said clostridial derivative is administered once every four weeks, five weeks, six weeks, seven weeks, eight weeks, nine weeks, ten weeks, eleven weeks, twelve weeks, thirteen weeks, fourteen weeks, fifteen weeks, sixteen weeks, seventeen weeks, eighteen weeks, nineteen weeks, twenty weeks, twenty one weeks, twenty two weeks, twenty three weeks, twenty four weeks, twenty five weeks, twenty six weeks or more.
